(19) Europäisches Patentamt
European Patent Office
Office européen des brevets

(11) Veröffentlichungsnummer : **0 576 396 A1**

(12) # EUROPÄISCHE PATENTANMELDUNG

(21) Anmeldenummer : **93810423.9**

(51) Int. Cl.$^5$ : **C07F 9/38**

(22) Anmeldetag : **14.06.93**

(30) Priorität : **22.06.92 CH 1984/92**

(43) Veröffentlichungstag der Anmeldung : **29.12.93 Patentblatt 93/52**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Jaeggi, Knut A., Dr.**
**General Guisan-Strasse 44**
**CH-4054 Basel (CH)**

(54) **Amidinverbindungen und neue Amidine und deren Verwendung zur Behandlung von Störungen des Calcium-Stoffwechsels.**

(57) Verbindungen der Formel I,

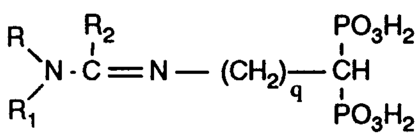

(I)

worin R, R1, R2 und q die in der Beschreibung angegebenen Bedeutungen haben, weisen wertvolle pharmazeutische Eigenschaften auf und sind insbesondere als Regulatoren des Calciumstoffwechsels wirksam. Sie werden in an sich bekannter Weise hergestellt.

EP 0 576 396 A1

Die Erfindung betrifft pharmazeutische Präparate enthaltend eine Verbindung der Formel I,

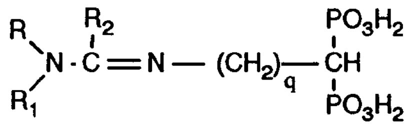

(I)

worin R, $R_1$ und $R_2$ unabhängig voneinander Wasserstoff oder Niederalkyl bedeuten; oder R und $R_1$ zusammen für einen bivalenten Rest $-(CH_2)_m$ - X - $(CH_2)_n$- stehen, (a) worin m und n unabhängig voneinander 1 bis 6 bedeuten, (b) worin die Summe aus m und n 2 bis 7 beträgt, und (c) worin X für eine Gruppe $-CH_2-$, -CH=CH-, -O-, $-N(R_3)-$ oder $-S(O)_p-$ steht, wobei $R_3$ Wasserstoff, Niederalkyl, Phenylniederalkyl oder Niederalkanoyl bedeutet und p für 0 bis 2 steht; und q für 0 bis 6 steht; oder ein pharmazeutisch anwendbares Salz davon, und mindestens ein pharmazeutisch verwendbares Trägermaterial, ferner neue Verbindungen der Formel I, Verfahren zur Herstellung letzterer, und die Verwendung der Verbindungen der Formel I zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Phenylniederalkyl ist z.B. Benzyl oder 2-Phenylethyl.

Halogen steht insbesondere für Chlor und Brom, kann aber auch Fluor oder Iod bedeuten.

Niederalkanoyl ist z.B. Formyl, Acetyl, Propionyl oder Pivaloyl.

Beispiele für die Gruppe $-NRR_1$, worin R und $R_1$ zusammen einen bivalenten Rest $-(CH_2)_m$ - X - $(CH_2)_n$- mit X = $-CH_2-$ bilden, sind Piperidino oder Pyrrolidino.

Beispiele für die Gruppe $-NRR_1$, worin R und $R_1$ zusammen einen bivalenten Rest $-(CH_2)_m$ - X - $(CH_2)_n$- mit X = -CH=CH- bilden, sind Dihydro-1H-pyrrol-1-yl oder 1,2,5,6-Tetrahydropyridin-1-yl.

Beispiele für die Gruppe $-NRR_1$, worin R und $R_1$ zusammen einen bivalenten Rest $-(CH_2)_m$ - X - $(CH_2)_n$- mit X = -O- bilden, sind Morpholino oder Hexahydro-3-oxa-1H-azepin-1-yl.

Beispiele für die Gruppe $-NRR_1$, worin R und $R_1$ zusammen einen bivalenten Rest $-(CH_2)_m$ - X - $(CH_2)_n$- mit X = $-N(R_3)-$ bilden, sind Piperazino, 4-Methylpiperazino, 4-Benzylpiperazino oder 4-Acetylpiperazino.

Beispiele für die Gruppe $-NRR_1$, worin R und $R_1$ zusammen einen bivalenten Rest $-(CH_2)_m$ - X - $(CH_2)_n$- mit X = $-S(O)_p-$ bilden, sind Thiomorpholino, S-Oxo-thio-morpholino oder S,S-Dioxothiomorpholino.

Salze von Verbindungen der Formel I sind z.B. deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Äthyl- oder Diäthylamin, Mono-, Di- oder Tri-(hydroxyniederalkyl)aminen, wie Äthanol-, Diäthanol- oder Triäthanolamin, Tris-(hydroxymethyl)methylamin oder 2-Hydroxytertiärbutylamin, oder N-(Hydroxyniederalkyl)-N,N-diniederalkyl-aminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. Tetrabutylammoniumhydroxid. Umfasst sind sowohl vollständige als auch partielle Salze, d.h. z.B. Salze mit 1, 2, 3 oder 4, vorzugsweise 2, Äquivalenten Base pro Mol Säure der Formel I.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. So bewirken sie an der Ratte eine ausgeprägte Hemmung der Knochenresorption, die sich sowohl in der Versuchsanordnung gemäss Acta Endocrinol. 78, 613-24 (1975) als auch anhand des PTH- induzierten Anstieges des Serumcalciumspiegels nach subkutaner Applikation in Dosen von etwa 0,01 bis etwa 1.0 mg/kg, als auch im TPTX-(Thyroparathyroidectomised)-Rattenmodell anhand der durch Vitamin $D_3$ ausgelösten experimentellen Hyperkalzämie nach Gabe von Dosen von etwa 0,0005 bis etwa 0,5 mg/kg s.c. und teilweise auch p.o. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis

der Ratte in der Versuchsanordnung nach Newbold, Brit. J. Pharmacology 21, 127 (1963) sowie nach Kaibara et al., J. Exp. Med. 159, 1388-96 (1984) in Dosen von etwa 0,01 bis etwa 1,0 mg/kg s.c. eine deutliche Hemmung des Fortschreitens chronisch-arthritischer Prozesse.

Die Verbindungen der Formel I und ihre Salze sind deshalb vorzüglich geeignet als Arzneimittelwirkstoffe z.B. für die Behandlung von Erkrankungen, die mit Störungen des Calcium-Stoffwechsels in Verbindung gebracht werden können, insbesondere tumorinduzierte Hyperkalzämie, Knochenmetastasen und Morbus Paget, sowie für die Behandlung von entzündlichen Prozessen in Gelenken, degenerativen Prozessen im Gelenkknorpel, Osteoporose, Periodontitis, Hyperparathyreoidismus, und ferner z.B. für die Behandlung von Calciumablagerungen in Blutgefässen oder in prothetischen Implantaten.

Hervorzuheben unter den oben erwähnten pharmazeutischen Präparaten enthaltend eine Verbindung der Formel I sind insbesondere diejenigen, die eine Verbindung der Formel I, worin R und $R_1$ unabhängig voneinander Niederalkyl bedeuten, oder R und $R_1$ zusammen für einen bivalenten Rest $-(CH_2)_m - X - (CH_2)_n-$ wie oben definiert stehen, und $R_2$ Wasserstoff oder Niederalkyl bedeutet, oder ein pharmazeutisch anwendbares Salz davon, enthalten.

Bevorzugt betrifft die Erfindung pharmazeutische Präparate enthaltend eine Verbindung der Formel I, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Dihydro-1H-pyrrol-1-yl, 1,2,5,6-Tetrahydropyridin-1-yl, Morpholino, Hexahydro-3-oxa-1H-azepin-1-yl, Piperazino, 4-Niederalkyl-piperazino, 4-Phenylniederalkyl-piperazino, 4-Niederalkanoyl-piperazino, Thiomorpholino, S-Oxo-thiomorpholino oder S,S-Dioxothiomorpholino bilden, $R_2$ Wasserstoff oder Niederalkyl bedeutet, und q für 0 bis 6 steht; oder ein pharmazeutisch anwendbares Salz davon, und mindestens ein pharmazeutisch verwendbares Trägermaterial.

Besonders bevorzugt sind pharmazeutische Präparate enthaltend eine Verbindung der Formel I, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Morpholino, Piperazino oder Thiomorpholino bilden, $R_2$ Wasserstoff oder Niederalkyl bedeutet, und q für 0 steht; oder ein pharmazeutisch anwendbares Salz davon, und mindestens ein pharmazeutisch verwendbares Trägermaterial.

Insbesondere betrifft die Erfindung pharmazeutische Präparate enthaltend eine Verbindung der Formel I, worin R und $R_1$ unabhängig voneinander Methyl oder Ethyl bedeuten, $R_2$ Wasserstoff bedeutet, und q für 0 steht, z.B. die N,N-Dimethyl-formamidino-methan-bisphosphonsäure, oder ein pharmazeutisch anwendbares Salz davon, und mindestens ein pharmazeutisch verwendbares Trägermaterial.

Die Erfindung betrifft ferner neue Verbindungen der Formel I,

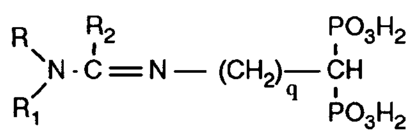

(I)

worin R, $R_1$ und $R_2$ unabhängig voneinander Wasserstoff oder Niederalkyl bedeuten; oder R und $R_1$ zusammen für einen bivalenten Rest $-(CH_2)_m - X - (CH_2)_n-$ stehen, (a) worin m und n unabhängig voneinander 1 bis 6 bedeuten, (b) worin die Summe aus m und n 2 bis 7 beträgt, und (c) worin X für eine Gruppe $-CH_2-$, $-CH=CH-$, $-O-$, $-N(R_3)-$ oder $-S(O)_p-$ steht, wobei $R_3$ Wasserstoff, Niederalkyl, Phenylniederalkyl oder Niederalkanoyl bedeutet und p für 0 bis 2 steht; und q für 0 bis 6 steht; mit der Massgabe, dass $R_2$ von Wasserstoff verschieden ist, wenn q für 0 steht und beide Reste R und $R_1$ unabhängig voneinander Wasserstoff, Methyl oder Ethyl bedeuten; oder Salze davon.

Hervorzuheben unter den oben erwähnten Verbindungen der Formel I sind insbesondere diejenigen, worin R und $R_1$ unabhängig voneinander Niederalkyl bedeuten, oder R und $R_1$ zusammen für einen bivalenten Rest $-(CH_2)_m- X - (CH_2)_n-$ wie oben definiert stehen, und $R_2$ Wasserstoff oder Niederalkyl bedeutet, und Salze davon.

Bevorzugt betrifft die Erfindung die Verbindungen der Formel I, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Dihydro-1H-pyrrol-1-yl, 1,2,5,6-Tetrahydropyridin-1-yl, Morpholino, Hexahydro-3-oxa-1H-azepin-1-yl, Piperazino, 4-Niederalkyl-piperazino, 4-Phenylniederalkyl-piperazino, 4-Niederalkanoyl-piperazino, Thiomorpholino, S-Oxo-thiomorpholino oder S,S-Dioxothiomorpholino bilden, $R_2$ Wasserstoff oder Niederalkyl bedeutet, und q für 0 bis 6 steht; mit der Massgabe, dass $R_2$ von Wasserstoff verschieden ist, wenn q für 0 steht und beide Reste R und $R_1$ unabhängig voneinander Methyl oder Ethyl bedeuten; oder ein pharmazeutisch anwendbares Salz davon.

Besonders bevorzugt sind die Verbindungen der Formel I, worin R $C_1$-$C_7$-Alkyl und $R_1$ $C_3$-$C_7$-Alkyl bedeuten; oder R und $R_1$ zusammen mit dem mit ihnen verknüpften Stickstoffatom für die Gruppe Piperidino, Pyrrolidino, Morpholino, Piperazino oder Thiomorpholino stehen, $R_2$ Wasserstoff oder Niederalkyl bedeutet, und q für 0 steht; oder ein pharmazeutisch anwendbares Salz davon.

Die Erfindung betrifft vor allem die in den Beispielen beschriebenen spezifischen Verbindungen und Salze davon.

Die neuen Verbindungen der Formel I können in an sich bekannter Weise hergestellt werden, indem man z.B.

a) in einer Verbindung der Formel II

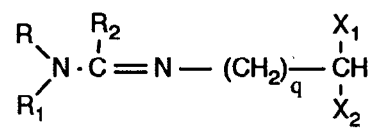

(II)

worin R, $R_1$, $R_2$ und q die unter Formel I angegebene Bedeutung haben, $X_1$ eine funktionell abgewandelte und $X_2$ eine freie oder funktionell abgewandelte Phosphonogruppe bedeutet, funktionell abgewandeltes Phosphono $X_1$ und gegebenenfalls $X_2$ in die freie Phosphonogruppe überführt, oder

b) eine Verbindung der Formel III,

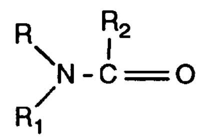

(III)

worin R, $R_1$ und $R_2$ wie unter Formel I definiert sind, oder ein funktionelles Derivat davon, mit einer Verbindung der Formel IV,

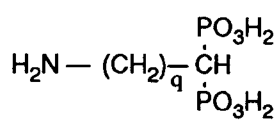

(IV)

worin q wie unter Formel I definiert ist, oder einem geeigneten Salz davon, umsetzt; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

In der folgenden näheren Beschreibung der Verfahren a)-b) haben die Symbole R, $R_1$, $R_2$ und q jeweils die unter Formel I angegebene Bedeutung, sofern nichts anderes angegeben ist.

Gemäss der Verfahrensvariante a) in Phosphono zu überführende funktionell abgewandelte Phosphonogruppen liegen beispielsweise in Esterform, insbesondere einer Diesterform der Formel $-P(=O)(OR)_2$ (IIa), vor, worin OR veräthertes Hydroxy, insbesondere Niederalkoxy, Niederalkanoyloxyniederalkoxy oder eine gegebenenfalls durch Niederalkyl, Niederalkoxy, Halogen, Trifluormethyl und/oder Hydroxy substituierte Phenoxy- oder $\alpha$-Phenylniederalkoxygruppe oder Silyloxy, wie Triniederalkylsilyloxy, bedeutet.

Die Überführung von funktionell abgewandelten in freie Phosphonogruppen erfolgt in üblicher Weise, wie durch Hydrolyse, beispielsweise in Gegenwart einer Mineralsäure, wie Salz- oder Schwefelsäure, bei etwa 80°C bis etwa 110°C, z.B. in der Siedehitze, oder durch Umsetzung mit einem Triniederalkylhalogensilan, z.B. mit Trimethylchlorsilan oder insbesondere Trimethyljodsilan oder Trimethylbromsilan, vorzugsweise in Methylenchlorid im Temperaturbereich von etwa 0°C bis etwa 40°C, und anschliessende Behandlung mit Wasser. $\alpha$-Phenylniederalkylester können ferner durch Hydrogenolyse, beispielsweise Umsetzung mit Wasserstoff in Gegenwart eines Hydrierungskatalysators, wie eines Nickel- oder Edelmetallkatalysators, z.B. von Palladium

auf Kohle, vorzugsweise in einem Niederalkanol unter normalen Temperatur- und Druckbedingungen, in Verbindungen der Formel I überführt werden.

Die Ausgangsstoffe der Formel II können beispielsweise hergestellt werden, indem man eine Verbindung der Formel III mit einer Verbindung der Formel V

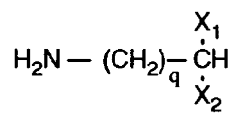

(V)

umsetzt, worin $X_1$ eine funktionell abgewandelte und $X_2$ eine freie oder eine funktionell abgewandelte Phosphonogruppe bedeutet.

Verfahren (b): Vorzugsweise wird ein funktionelles Derivat einer Verbindung der Formel III eingesetzt.

Ein funktionelles Derivat einer Verbindung der Formel III ist z.B. ein Acetal, vorzugsweise ein Diniederalkylacetal oder Niederalkylenacetal, insbesondere ein Dimethylacetal.

Die Umsetzung gemäss Verfahren (b) kann in Gegenwart eines Kondensationsmittels, z.B. $POCl_3$, oder auch ohne Kondensationsmittel durchgeführt werden.

Verfahrensgemäss erhältliche Verbindungen der Formel I können in üblicher Weise auch in andere Verbindungen der Formel I überführt werden.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Die neuen Verbindungen können, je nach Wahl der Ausgangsstoffe und Arbeitsweisen, in Form eines der möglichen Isomeren, z.B. je nach Anzahl der asymmetrischen Kohlenstoffatome als optische Isomere, wie in Form eines Enantiomeren, wie Antipoden, bzw. Diastereomeren, oder als Gemische derselben, wie Enantiomerengemische, z.B. Racemate, Diastereomerengemische oder Racematgemische, vorliegen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Ferner können erhaltene salzbildende Verbindungen in an sich bekannter Weise in Salze überführt werden, z.B. durch Umsetzung einer Lösung der freien Verbindung in einem geeigneten Lösungsmittel oder Lösungsmittelgemisch mit einer entsprechenden Base oder mit einem geeigneten Ionenaustauscher.

Erhaltene Salze können in an sich bekannter Weise in die freien Verbindungen umgewandelt werden, z.B. durch Behandeln mit einer Säure, wie einer Mineralsäure, z.B. mit Salzsäure.

Erhaltene Salze können in an sich bekannter Weise in andere Salze überführt werden, z.B. durch Behandeln mit einer geeigneten Base, wie Natriumhydroxid oder Kaliumhydroxid, Ammoniak oder einem geeigneten Amin.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Verbindungen der Formel I, einschliesslich ihrer Salze, können auch in Form von Hydraten erhalten werden oder das zur Kristallisation verwendete Lösungsmittel einschliessen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die erfindungsgemässen pharmazeutischen Präparate enthalten eine therapeutisch wirksame Menge der Aktivsubstanz, gegebenenfalls zusammen mit anorganischen oder organischen, festen oder flüssigen, pharmazeutisch verwendbaren Trägerstoffen, die sich z.B. zur enteralen, z.B. oralen, parenteralen oder transdermalen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsilikat, Starken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und-/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. Mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Zur parenteralen Verabreichung eignen sich in erster Linie wässrige Lösungen des Wirkstoffes in wasserlöslicher Form, z.B. in Form eines wasserlöslichen pharmazeutisch verwendbaren Salzes, ferner Suspensionen das Wirkstoffes, wie ölige Injektionssuspensionen, wobei man geeignete lipophile Lösungsmittel oder Vehikel, wie fette Öle, z.B. Sesamöl, oder synthetische Fettsäureester, z.B. Äthyloleat, ferner Triglyceride, verwendet oder wässrige Injektionssuspensionen, welche viskositätserhöhende Stoffe, z.B. Natriumcarboxymethylcellulose, Sorbit und/oder Dextran, und gegebenenfalls Stabilisatoren enthalten.

Geeignete Formulierungen für die transdermale Anwendung enthalten eine wirksame Menge einer erfindungsgemässen Verbindung mit einem Trägermittel. Vorteilhafte Trägermittel umfassen absorbierbare pharmakologisch annehmbare Lösungsmittel, um die Passage durch die Haut des Wirts zu erleichtern. Ueblicherweise haben transdermale Einrichtungen die Form einer Bandage, die (a) eine Stütze, (b) ein die Verbindung gegebenenfalls zusammen mit Trägermitteln enthaltendes Vorratsbehältnis, (c) gegebenenfalls eine Trenneinrichtung, die die Verbindung in einer gesteuerten und festgelegten Geschwindigkeit über einen langeren Zeitraum auf die Haut des Wirts abgibt, sowie (d) Mittel zur Befestigung der Einrichtung auf der Haut umfasst.

Die Erfindung betrifft weiterhin die Verwendung von Verbindungen der Formel I, zur Behandlung von auf Störungen des Calciumstoffwechsels zurückzuführenden Erkrankungen, vorzugsweise durch Bereitstellung von pharmazeutischen Präparaten. Die Dosierung der erfindungsgemässen Verbindung der Formel I kann von verschiedenen Faktoren, wie Applikationsweise, Spezies, Alter und/oder individuellem Zustand abhängen. Einzeldosen enthalten beispielsweise von etwa 0,01 bis etwa 0,1 mg, vorzugsweise 0,02 bis 0,08 mg bei parenteraler und etwa 0,2 bis etwa 2,5 mg, vorzugsweise 0,3 bis 1,5 mg, bei oraler Gabe jeweils pro Kilogramm Körpergewicht. Die bevorzugten Einzeldosen betragen somit etwa 0.5 bis 5.0 mg bei parenteraler und etwa 10 bis 100 mg bei oraler Applikation. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Drucke sind in mbar angegeben.

<u>Beispiel 1:</u> 3,9 g (0,0097 Mol) roher N-Butyl-N-methyl-formamidino-methan-bisphosphonsäure-tetraäthylester werden in 30 ml Methylenchlorid gelöst und bei 0°C mit 6,3 ml (0,049 Mol) Trimethylbromsilan versetzt. Nach 72 Stunden Stehen bei Raumtemperatur wird das Lösungsmittel unter vermindertem Druck entfernt. Das zurückbleibende Öl wird in 95%-igem wässrigen Methanol unter Erwärmen gelöst. Beim Erkalten scheidet sich die N-Butyl-N-methyl-formamidinomethan-bisphosphonsäure in farblosen Kristallen vom Smp. 200-201°C (Zers.) ab.

Die Ausgangsverbindung wird wie folgt hergestellt:

3,03 g (0,01 Mol) Amino-methylen-bisphosphonsäure-tetraäthylester werden mit 1,61 g (0,01 Mol) N-Butyl-N-methyl-formamid-dimethylacetal in 30 ml Tetrahydrofuran 5 Stunden unter Rückfluss gerührt. Nach Abdestillieren des Lösungsmittels erhält man den rohen N-Butyl-N-methyl-formamidino-methan-bisphosphonsäure-tetraäthylester, der ohne Reinigung weiterverwendet wird.

<u>Beispiel 2:</u> Analog zu Beispiel 1, jedoch bei der Herstellung der Ausgangsverbindung ausgehend von N-Butyl-N-methyl-acetamid-dimethylacetal anstelle von N-Butyl-N-methyl-formamid-dimethylacetal, erhält man die N-Butyl-N-methyl-acetamidinomethan-bisphosphonsäure vom Smp. 209-211°C (Zers.).

Die Ausgangsverbindung wird wie folgt hergestellt:

6,1 g (0,07 Mol) n-Butyl-methylamin werden mit 9,3 g (0,07 Mol) N,N-Dimethylacetamid-dimethylacetal während 24 h unter Rückfluss bei 130°C Badtemperatur gerührt. Durch anschliessende Destillation unter Vakuum erhält man das N-Butyl-N-methylacetamid-dimethylacetal, Kp. 72-75°C/31 mbar.

Beispiel 3: 2,8 g (0,007 Mol) Piperidino-formamidinomethan-bisphosphonsäure-tetraäthylester werden in 25 ml Dichlormethan gelöst und mit 4,5 ml (0,035 Mol) Trimethylbromsilan versetzt. Nach 72 Stunden Stehen bei Raumtemperatur wird das Lösungsmittel unter vermindertem Druck abdestilliert. Das zurückbleibende Öl wird mit heissem 95%-igem wässrigen Methanol aufgenommen, worauf die Piperidino-formamidino-methan-bisphosphonsäure vom Smp. 235-236°C kristallisiert.

Das Ausgangsmaterial wird wie folgt hergestellt:

1,27 g (0,008 Mol) Piperidino-formamid-dimethylacetal [Liebigs Ann. 641 (1961) 1] und 2,43 g (0,008 Mol) Aminomethylen-bisphosphonsäure-tetraäthylester werden in 30 ml Tetrahydrofuran (THF) gelöst und während 6 Stunden unter Rückfluss erhitzt. Nach Abziehen des Lösungsmittels unter vermindertem Druck erhält man den rohen Piperidino-formamidinomethan-bisphosphonsäure-tetraäthylester, der ohne Reinigung weiterverwendet wird.

Beispiel 4: In analoger Weise wie in den Beispielen 1-3 beschrieben können ferner hergestellt werden:

(a) Pyrrolidino-formamidinomethan-bisphosphonsäure, und

(b) 2-(N,N-Dimethyl-formamidino)-ethan-1,1-bisphosphonsäure.

Beispiel 5: 3,0 g (0,008 Mol) Pyrrolidino-formamidinomethan-bisphosphonsäure-tetraäthylester werden in 25 ml Dichlormethan gelöst und mit 4,5 ml (0,035 Mol) Trimethylbromsilan versetzt. Nach 72 Stunden Stehen bei Raumtemperatur wird das Lösungsmittel unter vermindertem Druck abdestilliert. Das zurückbleibende Öl wird mit heissem 95%-igem wässrigen Methanol aufgenommen, worauf die Pyrrolidino-formamidino-methan-bisphosphonsäure als Monohydrat vom Smp. 213-214°C (Zers.) kristallisiert.

Das Ausgangsmaterial wird wie folgt hergestellt:

2,43 g (0,008 Mol) Aminomethylen-bisphosphonsäure-tetraäthylester und 1,20 g (0,0082 Mol) 1-(Dimethoxymethyl)-pyrrolidin werden in 30 ml THF gelöst und während 40h unter Rückfluss zum Sieden erhitzt. Nach Abziehen des Lösungsmittels unter vermindertem Druck erhält man den rohen Pyrrolidino-formamidinomethan-bisphosphonsäure-tetraäthylester, der ohne Reinigung weiterverwendet wird.

Beispiel 6: Tabletten, enthaltend je 50 mg Wirkstoff, z.B. N,N-Dimethyl-formamidinomethan-bisphosphonsäure, oder ein Salz, z.B. das Dinatriumsalz, davon, können wie folgt hergestellt werden:

Zusammensetzung (10000 Tabletten)

| | |
|---|---|
| Wirkstoff | 500,0 g |
| Lactose | 500,0 g |
| Kartoffelstärke | 325,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, den Talk, das Magnesiumstearat und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 7: Lacktabletten, enthaltend je 100 mg Wirkstoff, z.B. N-Butyl-N-methyl-formamidinomethan-bisphosphonsäure oder ein Salz, z.B. das Dinatriumsalz, davon, können wie folgt hergestellt werden:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 8: Gelatinesteckkapseln, enthaltend 100 mg Wirkstoff, z.B. N-Butyl-N-methyl-acetamidinomethan-bisphosphonsäure oder ein Salz, z.B. das Dinatriumsalz, davon, können z.B. folgendermassen hergestellt werden:

| Zusammensetzung (für 1000 Kapseln) | |
|---|---|
| Wirkstoff | 100,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird die mikrokristalline Zellulose durch ein Sieb mit einer Maschenweite von 0,9 mm hinzugesiebt. Dann wird erneut 10 Minuten innig vermischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

Beispiel 9: Eine 0,2%-ige Injektions- oder Infusionslösung von Piperidino-formamidinomethan-bisphosphonsäure, oder einem Salz, z.B. dem Dinatriumsalz, davon, kann z.B. wie folgt hergestellt werden:

| Zusammensetzung (für 1000 Ampullen) | |
|---|---|
| Wirkstoff | 5,0 g |
| Natriumchlorid | 22,5 g |
| Phosphatpuffer pH=7.4 | 300,0 g |
| entmineralisiertes Wasser | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glas- oder Kunststoffampullen abgefüllt, die dann je 2,0 bzw. 5,0 mg Wirkstoff enthalten.

Beispiel 10: In analoger Weise wie in den Beispielen 6 bis 9 beschrieben kann man auch pharmazeutische

Präparate, enthaltend eine andere Verbindung der Formel I gemäss einem der Beispiele 1 bis 5 herstellen.

## Patentansprüche

1. Pharmazeutische Präparate enthaltend eine Verbindung der Formel I,

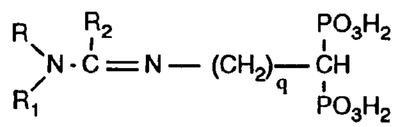

(I)

worin R, $R_1$ und $R_2$ unabhängig voneinander Wasserstoff oder Niederalkyl bedeuten; oder R und $R_1$ zusammen für einen bivalenten Rest $-(CH_2)_m-X-(CH_2)_n-$ stehen, (a) worin m und n unabhängig voneinander 1 bis 6 bedeuten, (b) worin die Summe aus m und n 2 bis 7 beträgt, und (c) worin X für eine Gruppe $-CH_2-$, $-CH{=}CH-$, -O-, $-N(R_3)-$ oder $-S(O)_p-$ steht, wobei $R_3$ Wasserstoff, Niederalkyl, Phenylniederalkyl oder Niederalkanoyl bedeutet und p für 0 bis 2 steht; und q für 0 bis 6 steht; oder ein pharmazeutisch anwendbares Salz davon, und mindestens ein pharmazeutisch verwendbares Trägermaterial.

2. Pharmazeutische Präparate gemäss Anspruch 1 enthaltend eine Verbindung der Formel I, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Dihydro- 1 H-pyrrol- 1 -yl, 1,2,5,6-Tetrahydropyridin-1-yl, Morpholino, Hexahydro-3-oxa-1H-azepin-1-yl, Piperazino, 4-Niederalkyl-piperazino, 4-Phenylniederalkyl-piperazino, 4-Niederalkanoyl-piperazino, Thiomorpholino, S-Oxo-thiomorpholino oder S,S-Dioxothiomorpholino bilden, $R_2$ Wasserstoff oder Niederalkyl bedeutet, und q für 0 bis 6 steht; oder ein pharmazeutisch anwendbares Salz davon, und mindestens ein pharmazeutisch verwendbares Trägermaterial.

3. Pharmazeutische Präparate gemäss Anspruch 1 enthaltend eine Verbindung der Formel I, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Morpholino, Piperazino oder Thiomorpholino bilden, $R_2$ Wasserstoff oder Niederalkyl bedeutet, und q für 0 steht; oder ein pharmazeutisch anwendbares Salz davon, und mindestens ein pharmazeutisch verwendbares Trägermaterial.

4. Pharmazeutische Präparate gemäss Anspruch 1 enthaltend eine Verbindung der Formel I, worin R und $R_1$ unabhängig voneinander Methyl oder Ethyl bedeuten, $R_2$ Wasserstoff bedeutet, und q für 0 steht, oder ein pharmazeutisch anwendbares Salz davon, und mindestens ein pharmazeutisch verwendbares Trägermaterial.

5. Pharmazeutische Präparate gemäss Anspruch 1 enthaltend N,N-Dimethyl-formamidinomethan-bisphosphonsäure oder ein pharmazeutisch anwendbares Salz davon, und mindestens ein pharmazeutisch verwendbares Trägermaterial.

6. Pharmazeutische Präparate gemäss Anspruch 1 enthaltend Piperidino-formamidinomethanbisphosphonsäure oder ein pharmazeutisch anwendbares Salz davon, und mindestens ein pharmazeutisch verwendbares Trägermaterial.

7. Verbindungen der Formel I,

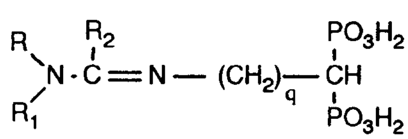

(I)

worin R, $R_1$ und $R_2$ unabhängig voneinander Wasserstoff oder Niederalkyl bedeuten; oder R und $R_1$ zusammen für einen bivalenten Rest $-(CH_2)_m-X-(CH_2)_n-$ stehen, (a) worin m und n unabhängig voneinander 1 bis 6 bedeuten, (b) worin die Summe aus m und n 2 bis 7 beträgt, und (c) worin X für eine Gruppe $-CH_2-$,

-CH=CH-, -O-, -N($R_3$)- oder -S(O)$_p$- steht, wobei $R_3$ Wasserstoff, Niederalkyl, Phenylniederalkyl oder Niederalkanoyl bedeutet und p für 0 bis 2 steht; und q für 0 bis 6 steht; mit der Massgabe, dass $R_2$ von Wasserstoff verschieden ist, wenn q für 0 steht und beide Reste R und $R_1$ unabhängig voneinander Wasserstoff, Methyl oder Ethyl bedeuten; oder Salze davon.

**8.** Verbindungen der Formel I gemäss Anspruch 7, worin R und $R_1$ unabhängig voneinander Niederalkyl bedeuten, oder R und $R_1$ zusammen mit dem mit ihnen verknüpften Stickstoffatom die Gruppe Piperidino, Pyrrolidino, Dihydro-1H-pyrrol-1-yl, 1,2,5,6-Tetrahydropyridin-1-yl, Morpholino, Hexahydro-3-oxa-1H-azepin-1-yl, Piperazino, 4-Niederalkyl-piperazino, 4-Phenylniederalkyl-piperazino, 4-Niederalkanoyl-piperazino, Thiomorpholino, S-Oxo-thiomorpholino oder S,S-Dioxothiomorpholino bilden, $R_2$ Wasserstoff oder Niederalkyl bedeutet, und q für 0 bis 6 steht; mit der Massgabe, dass $R_2$ von Wasserstoff verschieden ist, wenn q für 0 steht und beide Reste R und $R_1$ unabhängig voneinander Methyl oder Ethyl bedeuten; oder ein pharmazeutisch anwendbares Salz davon.

**9.** Verbindungen der Formel I gemäss Anspruch 7, worin R $C_1$-$C_7$-Alkyl und $R_1$ $C_3$-$C_7$-Alkyl bedeuten; oder R und $R_1$ zusammen mit dem mit ihnen verknüpften Stickstoffatom für die Gruppe Piperidino, Pyrrolidino, Morpholino, Piperazino oder Thiomorpholino stehen, $R_2$ Wasserstoff oder Niederalkyl bedeutet, und q für 0 steht; oder ein pharmazeutisch anwendbares Salz davon.

**10.** Piperidino-formamidinomethan-bisphosphonsäure gemäss Anspruch 7, oder ein pharmazeutisch anwendbares Salz davon.

**11.** Pharmazeutische Präparate enthaltend eine Verbindung gemäss einem der Ansprüche 7-10 und mindestens ein pharmazeutisch verwendbares Trägermaterial.

**12.** Eine Verbindung gemäss einem der Ansprüche 7-10 zur Anwendung in einem Verfahren zur therapeutischen Behandlung des tierischen oder menschlichen Körpers.

**13.** Eine Verbindung gemäss einem der Ansprüche 7-10 zur Verwendung bei der Behandlung von Krankheiten, die mit Störungen des Calcium-Stoffwechsels in Verbindung stehen.

**14.** Verwendung einer Verbindung gemäss einem der Ansprüche 7-10 zur Herstellung pharmazeutischer Präparate.

**15.** Verwendung einer Verbindung gemass einem der Ansprüche 7-10 zur Herstellung pharmazeutischer Präparate für die Behandlung von Krankheiten, die mit Störungen des Calcium-Stoffwechsels in Verbindung stehen.

**16.** Verfahren zur Herstellung einer Verbindung der Formel I gemäss Anspruch 7, dadurch gekennzeichnet, dass man

a) in einer Verbindung der Formel II

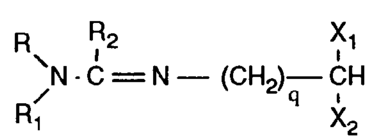

(II)

worin R, $R_1$, $R_2$ und q die unter Formel I angegebene Bedeutung haben, $X_1$ eine funktionell abgewandelte und $X_2$ eine freie oder funktionell abgewandelte Phosphonogruppe bedeutet, funktionell abgewandeltes Phosphono $X_1$ und gegebenenfalls $X_2$ in die freie Phosphonogruppe überführt, oder

b) eine Verbindung der Formel III,

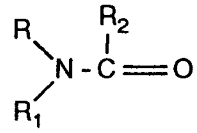

(III)

worin R, $R_1$ und $R_2$ wie unter Formel I definiert sind, oder ein funktionelles Derivat davon, mit einer Verbindung der Formel IV,

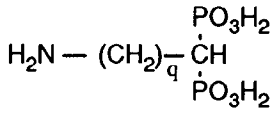

(IV)

worin q wie unter Formel I definiert ist, oder einem geeigneten Salz davon, umsetzt; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

Europäisches Patentamt

# EUROPÄISCHER RECHERCHENBERICHT

Nummer der Anmeldung

EP 93 81 0423

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-9 103 481 (BOEHRINGER MANNHEIM GMBH) 21. März 1991 * das ganze Dokument * --- | 1,7, 11-16 | C07F9/38 |
| A | DE-A-2 439 355 (JOH. A. BENCKISER GMBH.) 26. Februar 1976 * das ganze Dokument * --- | 1-16 | |
| P,X | EP-A-0 531 253 (CIBA-GEIGY AG) 10. März 1993 * das ganze Dokument * ----- | 1 | |
| | | | **RECHERCHIERTE SACHGEBIETE (Int. Cl.5 )** |
| | | | C07F |

Der vorliegende Recherchenbericht wurde für alle Patentansprüche erstellt

| Recherchenort | Abschlußdatum der Recherche | Prüfer |
|---|---|---|
| DEN HAAG | 24 SEPTEMBER 1993 | MAIR J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.....................................................
& : Mitglied der gleichen Patentfamilie, übereinstimmendes Dokument

EPO FORM 1503 03.82 (P0403)